## Europäisches Patentamt
## European Patent Office
## Office européen des brevets

(19)

(11) Veröffentlichungsnummer : **0 098 967**
**B1**

(12) **EUROPÄISCHE PATENTSCHRIFT**

(45) Veröffentlichungstag der Patentschrift :
09.08.89

(51) Int. Cl.⁴ : **C 12 N 15/00**, C 12 N 1/20// C12P21/02

(21) Anmeldenummer : 83105569.4

(22) Anmeldetag : 07.06.83

(54) **Verfahren zur Herstellung von obligat methylotrophen, Fremd-DNA exprimierenden Bakterien und dafür geeignete Plasmide und Wirtsorganismen.**

(30) Priorität : 11.06.82 DE 3222142
15.04.83 DE 3313643

(43) Veröffentlichungstag der Anmeldung :
25.01.84 Patentblatt 84/04

(45) Bekanntmachung des Hinweises auf die Patenterteilung : 09.08.89 Patentblatt 89/32

(84) Benannte Vertragsstaaten :
AT BE CH DE FR GB IT LI LU NL SE

(56) Entgegenhaltungen :
EP--A-- 0 035 831
EP--A-- 0 037 273
EP--A-- 0 062 971
EP--A-- 0 066 994
GB--A-- 2 003 926
NATURE, Band 297, Nr. 5861, Mai 1982, Seiten 80-82, Chesham, Bucks, GB; J.F. HENNAM et al.: "Expression of eukaryotic coding sequences in Methylophilus methylotrophus"
NATURE, vol. 287, no. 5781, Oktober 1980, Seiten 396-401, Chesham, Bucks, GB; J.D. WINDASS et al.: "Improved conversion of methanol to single-cell protein by Methylophilus methylotrophus"
CHEMICAL ABSTRACTS, Band 98, Nr. 3, 17. January 1983, Seite 296, Nr. 14119f, Columbus, Ohio, US; U. STAHL et al. "Plasmids in Methylomonas clara, a methylotrophic producer of single cell protein" & EUR. J. APPL. MICROBIOL. BIOTECHNOL. 1982, 15(4), 223-6

(73) Patentinhaber : HOECHST AKTIENGESELLSCHAFT
Postfach 80 03 20
D-6230 Frankfurt am Main 80 (DE)

(72) Erfinder : Winnacker, Ernst Ludwig, Prof. Dr.
Dall-Armistrasse 41a
D-8000 München 19 (DE)
Erfinder : Esser, Karl, Prof. Dr.
Am Spik 23a
D-4630 Bochum (DE)
Erfinder : Präve, Paul, Prof, Dr.
Pfarrstrasse 5
D-6232 Bad Soden am Taunus (DE)
Erfinder : Stahl, Ulf, Dr.
Biermannsweg 22
D-4630 Bochum (DE)
Erfinder : Marquardt, Rüdiger, Dr.
Belfortstrasse 1
D-8000 München 80 (DE)
Erfinder : Wöhner, Gerhard, Dr.
Flörsheimer Strasse 27
D-6093 Flörsheim am Main (DE)

EP 0 098 967 B1

# EP 0 098 967 B1

**Beschreibung**

Die Erfindung betrifft die genetische Manipulation von obligat methylotrophen Mikroorganismen, zur Herstellung von obligat methylotrophen Bakterien, die enthaltene Fremd-DNA exprimieren, Plasmide zur Einführung der Fremd-DNA und Wirtsorganismen. Das erfindungsgemäße Verfahren ist dadurch gekennzeichnet, daß man

a) ein aus einem obligat methylotrophen Bakterium stammendes Plasmid isoliert,

b) hieraus und aus einem Plasmid mit Selektionsmarkern ein Hybridplasmid mit einem dem obligat methylotrophen Bakterium eigenen Replicon herstellt,

c) dieses Hybridplasmid durch Transformation in einen Wirtsorganismus einbringt und dort amplifiziert,

d) nach Selektion die Klone mit einem geeigneten konjugativen Plasmid behandelt und den Mobilisierbarkeitsdefekt des Hybridplasmids behebt,

e) die so erhaltenen Klone mit obligat methylotrophen Bakterien als Rezipient konjugiert und

f) die gewünschten Klone selektioniert.

Das im Verfahrensschritt a) eingesetzte Plasmid wird vorzugsweise aus einem Bakterium der Gattung Methylomonas, insbesondere der Art Methylomonas clara isoliert. Besonders bevorzugt ist das Plasmid pBE 3 aus dem Methylomonas clara-Stamm, der bei der Deutschen Sammlung von Mikroorganismen unter der Nummer DSM 2397 hinterlegt ist, sowie entsprechende Plasmide mit demselben Replicon.

Das im Verfahrensschritt b) eingesetzte Plasmid mit Selektionsmarkern kann das Plasmid pBR 322 sein, das in Gene 2 (1977) 95-113 beschrieben ist. Die so erhaltenen Hydridplasmide werden im folgenden als $pRM_x$ bezeichnet. Gegebenenfalls können die Hybridplasmide auch die genetische Information für die Expression von Insulin enthalten, wie es in der europäischen Patentanmeldung 0 032 675 beschrieben ist.

Das nach Verfahrensschritt b) erhaltene Hybridplasmid wird dann durch Transformation in einen geeigneten Wirtsorganismus, vorteilhaft Escherichia coli, eingebracht und dort amplifiziert.

Im Verfahrensschritt d) wird in diesen Wirtsorganismus, der das Hybridplasmid enthält, ein geeignetes konjugatives Plasmid, vorteilhaft RP 4, eingebracht. Zusätzlich wird durch Einführen geeigneter Plasmide wie Col K und Col V der Mobilisierbarkeitsdefekt komplementiert.

Der so vorbereitete Wirtsorganismus kann dann im Verfahrensschritt e) das im Verfahrensschritt c) eingebrachte Hybridplasmid durch Konjugation auf ein vorzugsweise plasmidfreies obligat methylotrophes Bakterium als Rezipient übertragen. Als Rezipient bevorzugt sind Bakterien der Gattung Methylomonas, vorzugsweise von der Art Methylomonas clara, insbesondere vom Stamm ATCC 31226. Dieser Stamm ist beispielsweise in der Deutschen Patentschrift 2 633 451 und in der US-Patentschrift 4 166 004 beschrieben.

Abschließend werden im Verfahrensschritt f) die gewünschten Klone durch Anzucht in einem Medium, das Methanol als Kohlenstoffquelle enthält, sowie auf Grund der übertragenen Resistenz bzw. Empfindlichkeit gegenüber Antibiotika selektioniert. Die so erhaltenen obligat methylotrophen Bakterien sind in der Lage, die enthaltene Fremd-DNA zu exprimieren, also beispielsweise Insulin zu produzieren.

Einzelheiten dieser Verfahrensschritte werden im Rahmen der Beispiele näher ausgeführt.

Die Erfindung betrifft weiterhin eine Variante des vorstehend genannten Verfahrens, bei dem die Verfahrensschritte d) und e) wie folgt vereinfacht werden :

Im Schritt d) wird nach Selektion das Hybridplasmid isoliert und im Schritt e) das isolierte Plasmid durch Transformation in Sphäroplasten des obligat methylotrophen Bakteriums eingebracht.

Die Erfindung betrifft somit auch Sphäroplasten aus methylotrophen Bakterien, vor allem aus obligat methylotrophen Bakterien, insbesondere aus Bakterien der Gattung Methylomonas, vorzugsweise der Art Methylomonas clara. Besonders bevorzugt ist der Stamm Methylomonas clara ATCC 31226.

Die erfindungsgemäßen Sphäroplasten kann man dadurch herstellen, daß man die Bakterien in einem glycinreichen Medium, das einen osmotischen Stabilisator enthält, züchtet. Vorzugsweise ist dieses Medium schwach hypotonisch. Weitere bevorzugte Ausgestaltungen dieses Herstellungsverfahrens werden im folgenden näher erläutert.

Die Erfindung betrifft weiterhin die Verwendung der neuen Sphäroplasten zum Einbringen von externer DNA in diese Bakterien. Diese externe DNA wird in erster Linie in Form eines Plasmids eingebracht. Als Plasmide kommen die obengenannten Hybridplasmide in Betracht.

Zur Bildung der Späroplasten werden die methylotrophen Bakterien in einem geeigneten Medium, vorteilhaft in einem Minimalmedium, bis zu einer geeigneten Zelldichte angezüchtet. Geeignete Zelldichten liegen in einem Bereich der $OD_{600}$ von vorzugsweise 0,5 bis 1,8, insbesondere 0,9 bis 1,4. Ein definiertes Volumen dieser Kulturen wird dann in die etwa 5-fache Menge des genannten Mediums eingebracht, das außerdem reich an Glycin ist und einen osmotischen Stabilsator enthält. Der Gehalt an Glycin kann bis zur Sättigungskonzentration gehen, bevorzugt ist ein Gehalt von 2 bis 4 Gew.-%.

Als osmotische Stabilisatoren kommen Zucker wie Saccharose, Zuckeralkohole wie Sorbit und Polyglykole wie Polyethylenglykol 6 000 in Betracht. Das Medium ist vorszugsweise schwach hypotonisch, was durch geeignete Konzentrationen an osmotischem Stabilisator eingestellt wird. Geeignet ist beispielsweise eine Saccharosekonzentration von 10 Gew.-% oder einmolares Sorbit.

In diesem Medium werden die Bakterien solange geschüttelt, bis unter dem Phasen-Kontrastmikroskop im wesentlichen keine stäbchenförmigen methylotrophen Bakterien erkennbar sind. Im allgemeinen können nach etwa einer Stunde Schütteln (100 bis 180 Upm) bei etwa 37 °C die Bildung der Sphäroplasten als unbewegliche, kugelige Strukturen beobachtet werden. Dieser Prozeß ist im allgemeinen nach etwa 4 Stunden abgeschlossen.

Die gebildeten Späroplasten werden vorsichtig abzentrifugiert, beispielsweise bei 4 °C 10 Minuten bei 2 600 g, und in geeigneten Medien resuspendiert. Zur Stabilisierung der Sphäroplasten enthält dieses Resuspendierungsmedium ebenfalls einen osmotischen Stabilisator in geeigneter Konzentration, beispielsweise 15 Gew.-% Polyethylenglykol vom Molgewicht 6 000.

Diese Suspension wird in einem ungefähren Volumenverhältnis von 5 : 1 mit einer Mischung versetzt, die zu gleichen Teilen aus dem zur Resuspension der Sphäroplasten benutzten Medium und dem Träger der einzubringenden DNA in einem geeigneten Puffer besteht. Als DNA-Träger kommt — wie vorstehend ausgeführt — in erster Linie ein Hybridplasmid in Betracht. Ein geeigneter Puffer hierfür besteht aus einer wäßrigen Lösung, die pro Liter 10 mmol TRIS (Tris-(hydroxymethyl)-aminomethan) und 1 mmol Natrium-Ethylendiamintetraacetat (TE-Puffer, pH 8,0) enthält. Zu dieser Mischung wird ein osmotischer Stabilisator gegeben (beispielsweise das dreifache Volumen der Sphäroplasten-Suspension an 4 Gew.-%iger Polyethylenglykol 6 000-Lösung) und vorsichtig durchmischt. Nach kurzem Stehen bei Raumtemperatur wird etwa das 10-fache Volumen der Sphäroplasten-Suspension an Resuspensionsmedium zugegeben und die Mischung vorsichtig zentrifugiert (3 600 g).

Der Niederschlag wird dann in dem doppelten Volumen der Sphäroplasten-Suspension an Resuspensionsmedium aufgenommen und zur Überprüfung, ob die Transformation stattgefunden hat, auf Agarplatten ausgestrichen. Diese Agarplatten entsprechen in ihrer Zusammensetzung dem Resuspensionsmedium und enthalten neben 1,5 Gew.-% Bacto-Agar noch ein zur Selektion geeignetes Antibiotikum, beispielsweise 50 µg/ml Ampicillin oder 10 µg/ml Tetracyclin, falls die eingesetzten Hybridplasmide die entsprechenden Resistenzgene enthielten. Die Platten werden dann bei 37 °C für 1 bis 3 Tage bebrütet und resistente Kolonien nach Anzucht in dem genannten Flüssigmedium, das ein geeignetes Antibiotikum enthält, auf das Vorhandensein von Plasmid-DNA untersucht (Humphreys et al., BAA 383 (1975) 457-463). Hierbei können die zur Transformation eingesetzten Plasmide aus den methylotrophen Bakterien isoliert werden.

Gegenstand der Erfindung sind weiterhin Plasmide aus dem Methylomonas clara-Stamm DSM 2397 sowie die Hybridplasmide mit einem einem obligat methylotrophen Bakterium eigenen Replicon, also Plasmide, die in den Bakterien der Gattung Methylomonas, vorzugsweise der Art Methylomonas clara, insbesondere dem Stamm ATCC 31226 repliziert werden, in denen prokaryotische oder eukaryotische DNA integriert ist, insbesondere diejenige für die Expression von Insulin.

Gegenstand der Erfindung sind weiterhin die Wirtsorganismen, die die genannten Plasmide enthalten, sowie die Wirtsorganismen, die zusätzlich das konjugative Plasmid und weiterhin solche, die zusätzlich Plasmide zur Aufhebung des Mobilisierungsdefekts enthalten. Bevorzugte Wirtsorganismen enthalten als konjugatives Plasmid RP 4 und als Plasmide zur Aufhebung des Mobilisierungsdefekts Col K oder Col V.

Der Vorteil der Erfindung liegt darin, daß von einem Replicon eines Plasmids aus einem obligat methylotrophen Bakterium, also mit sehr engem Wirtsbereich, Gebrauch gemacht wird. Das erfindungsgemäße Verfahren zeichnet sich somit durch hohe Sicherheit aus, so daß es auf rekombinante DNA, die risikoreiche Informationen enthält, anwendbar ist.

In den folgenden Beispielen wird die Erfindung näher erläutert. Prozentangaben beziehen sich hierbei auf das Gewicht, sofern nichts anderes angegeben ist.

In den Beispielen wurden die folgenden Abkürzungen verwendet :

ATP = Adenosintriphosphat

EDTA = Ethylendiamin-tetraessigsäure bzw. -acetat (Na)

$OD_{600}$ = Optische Dichte (optical density) bei 600 nm

TE-Puffer = wäßrige Lösung, enthaltend pro Liter 10 m Mol Tris-HCl, auf pH 8 eingestellt, und 1 m Mol EDTA, ebenfalls auf pH 8,0 eingestellt

Tris (—HCl) = Tris-hydroxymethyl-aminomethan (-Hydrochlorid)

## Beispiele

### 1. Plasmid-Isolierung aus M. Clara DSM 2397

Die Plasmid-Isolierung erfolgte im wesentlichen nach der Methode von Humphreys et al. (BBA 383 (1975) 457-463). Hierzu wurden die Bakterien aus 1 l Kulturmedium bei einer $OD_{600}$ von etwa 1,0 abzentrifugiert und der Bakterienniederschlag in 5 ml Sucroselösung (25 % Sucrose in 50 mMol/l Tris-HCl Lösung vom pH 8,0) resuspendiert. Unter Eiskühlung wurden 1 ml Lysozymlösung (5 mg/ml Lysozym in 250 mMol/l Tris-HCl Lösung, pH 8,0) sowie 2 ml 0,2 molare EDTA-Lösung vom pH 8,0 zugegeben. Unter gelegentlichem Umschwenken wurde die Mischung 5 Minuten lang auf Eis inkubiert. Dann wurde die Lysis durch Zugabe von 8 ml einer Mischung, bestehend aus 50 mMol/l Tris-HCl, 75,5 mMol/l EDTA und 0,2 % eines nichtionischen Tensids (®Triton X-100) vom pH 8,0, herbeigeführt. Die hochviskose Mischung wurde 30 Minuten bei 48 000 g zentrifugiert, wobei als Überstand ein klares Lysat erhalten wurde. Pro 10 ml Überstand wurden 1,1 ml 5-molare Kochsalzlösung sowie 1,1 g Polyethylenglycol vom mittleren Molgewicht 6 000 zugegeben. Die Mischung wurde bei 4 °C über Nacht inkubiert. Der flockige Niederschlag wurde durch 5-minütige Zentrifugation bei 1 500 g gesammelt und in 3,5 ml Pufferlösung (50 mMol/l Tris-HCl, 5 mMol/l EDTA, 50 mMol/l NaCl, ph 8,0) gelöst und das Volumen der Lösung gemessen. Nach Zugabe von 1 g CsCl pro ml Lösung sowie von 1/15 ml einer 1 %igen wäßrigen Ethidiumbromid-Lösung wurde 10 Minuten bei 16 000 g zentrifugiert. Der Überstand dieser Zentrifugation wurde ins Gleichgewicht zentrifugiert (20 Stunden bei 47 000 Umdrehungen pro Minute, 18 °C, im Vertikalrotor) und wies auch ohne Bestrahlung mit UV-Licht zwei deutlich sichtbare, etwz einen Zentimeter auseinanderliegende Banden auf. Durch Einstrahlen von UV-Licht der Wellenlänge 366 nm wurden die fluoreszierenden Banden hervorgehoben und die untere Plasmid-Bande durch seitliches Anstechen des Gradienten mit einer Nadel und anschließendes Aufziehen der Bande in einer Spritze geerntet. Das Ethidiumbromid wurde durch wiederholtes Ausschütteln mit and CsCl gesättigtem Isopropanol entfernt. Nach 3 maliger Dialyse gegen 2 l TE-Puffer für jeweils mindestens 2 Stunden wurde die Plasmid-DNA in einer Reinheit erhalten, die den Einsatz in den folgenden Verfahrensschritten erlaubte.

Zeigten sich in der DNA-Lösung noch Verunreinigungen, so wurde diese zweimal mit gleichen Volumina an mit 0,1 molarer Tris-HCl Lösung vom pH 8,0 gesättigtem Phenol und dann 3 mal mit absolutem Ether extrahiert. Der überschüssige Ether wurde abgeblasen, die Lösung mit 1/9 des Volumens an dreimolarer Natriumacetatlösung versetzt und die DNA durch Zugabe des gleichen Volumens an Isopropanol gefällt. Nach Stehen über Nacht wurde die Mischung 30 Minuten lang bei 12 000 g zentrifugiert und der DNA-Niederschlag mit 90 %igem Ethanol gewaschen. Danach wurde die DNA lyophilisiert und mit TE-Puffer aufgenommen.

2. DNA-Isolierung aus Agarose

Im folgenden Verfahren fand die von Langridge et al. (Analytical Biochemistry 103 (1980) 264-271) beschriebene Methode Anwendung.

Die DNA wurde auf ein horizontales 0,5 %iges Agarose-Gel aufgetragen (niedrig schmelzende Agarose, Typ VII, No. A-4018, Sigma). Nach der Elektrophorese wurden die Banden durch Ethidiumbromid-Anfärbung sichtbar gemacht und aus dem Gel ausgeschnitten. Das die DNA enthaltende Agarosescheibchen wurde bei 70 °C in einem Glasröhrchen geschmolzen, das Volumen gemessen und auf 37 °C abgekühlt. Hierzu wurden gleiche Volumenmengen der nachstehend beschriebenen Butanol- und Wasserphasen zugegeben.

150 ml n-Butanol wurden mit 150 ml Wasser im Scheidetrichter geschüttelt und nach Trennung der Phasen in 100 ml der wassergesättigten Butanolphase 1 g Hexadecyl-trimethylamminiumbromid gelöst. Die Lösung wurde mit 100 ml der Wasserphase ausgeschüttelt, wobei ein Entschäumer zugegeben werden kann (50 µl Antifoam A, Sigma). Nach Trennung der Phasen über Nacht wurden diese getrennt aufgefangen.

Die nach Zugabe dieser Butanol- und Wasserphasen erhaltene Mischung wurde durch vorsichtiges Drehen des Röhrchens gründlich durchmischt und die Trennung der Phasen bei 37 °C abgewartet. Die obere, die DNA enthaltende Butanolphase wurde abgetrennt und die verbleibende wäßrige Phase noch zweimal in der beschriebenen Weise mit Butanol extrahiert. Die vereinigten Butanolphasen wurden mit 1/4 des Volumens an 0,2 molarer NaCl-Lösung versetzt und wieder gründlich durchgemischt. Nach Abtrennung der wäßrigen Phase erfolgte eine erneute Salzextraktion und die vereinigten wäßrigen Phasen wurden tropfenweise mit dem gleichen Volumen an Chloroform versetzt (das über eine Aluminiumoxidsäule gereinigt worden war). Nach halbstündigem Stehen auf Eis wurde die untere Chloroformphase verworfen und verbliebenes Chloroform mit Luft ausgeblasen. Die DNA wurde mit Isopropanol gefällt und nach Abtrennung in TE-Puffer resuspendiert.

3. Elektrophorese an Polyacrylamid-Gel

Zur Sichtbarmachung und Charakterisierung von DNA-Fragmenten unter 0,3 MD fanden 8-15 %ige Polyacrylamid-(PAA)-Gele Anwendung. Die Gele waren 1 mm dick, 30 cm lang und 14 cm breit. Als Puffer diente eine Mischung, die pro Liter 89 mMol Borsäure, 89 mMol Tris-HCl und 2,5 mMol EDTA enthielt und den pH 8,2 aufwies.

Zur Herstellung eines 8 %igen PAA-Gels wurden 33 ml einer 24 %igen PAA-Stocklösung (23,22 g Acrylamid und 0,78 g N,N'-Methylenbisacrylamid in 100 g wäßriger Lösung), 10 ml des genannten Elektrophoresepuffers, der jedoch alle Komponenten in 10-facher Konzentration enthält, 6,25 ml einer 6,4 %igen 3-Ethylamino-propionitril-Lösung und 50,75 ml Wasser zusammengegeben. Die Mischung wurde an der Wasserstrahlpumpe entgast und die Polymerisation mit festem· Ammoniumperoxodisulfat gestartet. Sofort anschließend wurde das Gel gegossen und bis zum Beginn der Elektrophorese mindestens 2 Stunden gewartet. Vor dem Auftragen der Proben wurde an das Gel zur Entfernung von restlichem Ammoniumperoxodisulfat etwa eine Stunde lang eine Spannung von 100 V angelegt.

Die Elektrophoresen wurden bei 100 V und einem Stromfluß von 12 mA durchgeführt.

4. Charakterisierung des Plasmids pBE 3 aus dem Methylomonas clara-Stamm DSM 2397

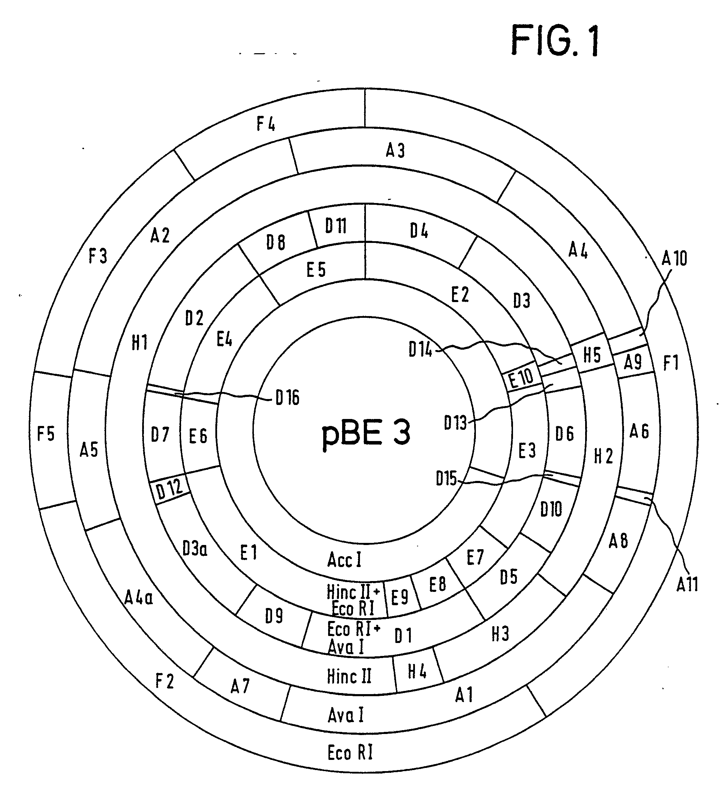

Das Plasmid pBE 3 wurde mit den in der Tabelle 1 genannten Restriktionsendonukleasen verdaut und die entstandenen Fragmente gelelektrophoretisch aufgetrennt. Durch Doppelverdauungen und durch Charakterisierung einzelner, in pBR 322 klonierter Fragmente — wie nachstehend beschrieben — wurden die in Tabelle 2 und Figur 1 niedergelegten Ergebnisse erhalten.

Das Plasmid pBR 3 ist eine Deletionsmutante aus einem größeren Plasmid. Daneben existieren noch kleinere Plasmide. Alle diese Plasmide sind im Sinne dieser Erfindung äquivalent, sofern sie das methylomonas-eigene Replicon enthalten.

Tabelle 1

Übersicht der zur Charakterisierung von pBE 3 verwendeten Enzyme :

| Enzym | Zahl der Fragmente |
|-------|-------------------:|
| Acc I | 1 |
| Xor II | 1 |
| Eco RI | 5 |
| Hinc II | 5 |
| Ava I | 12 |
| Bal I | mind. 19 |

Keine Spaltung mit
Bam HI
Bgl II
Bst EII
Eco RV
Hind III
Hpa I
Kpn I
Nru I
Pst I
Pvu II
Sal I
Sma I
Sph I
Sst I
Sst II
Stu I
Xba I
Xho I
Xmn I

Tablelle 2

Größe der Fragmente (in MD), die durch Spaltung von pBE 3 mit Restriktionsnucleasen erhalten wurden :

| EcoR I: | | Hinc II: | | Ava I: | |
|---------|------|----------|------|--------|------|
| F1 : | 4,14 | H1 : | 6,8 | A1 : | 2,0 |
| F2 : | 3,09 | H2 : | 1,42 | A2 : | 1,72 |
| F3 : | 1,27 | H3 : | 0,87 | A3 : | 1,26 |
| F4 : | 0,91 | H4 : | 0,31 | A4a: | 1,03 |
| F5 : | 0,63 | H5 : | 0,28 | A4 : | 1,03 |
| | | | | A5 : | 0,85 |
| | | | | A6 : | 0,66 |
| | | | | A7 : | 0,55 |
| | | | | A8 : | 0,50 |
| | | | | A9 : | 0,13 |
| | | | | A10: | 0,08 |
| | | | | A11: | 0,07 |

(Fortsetzung)

| EcoR I +<br>Ava I : | EcoR I +<br>Hinc II: |
|---|---|
| D1 : 1,35 | E1 : 2,29 |
| D2 : 1,22 | E2 : 1,83 |
| D3a: 1,03 | E3 : 1,42 |
| D3 : 1,03 | E4 : 1,27 |
| D4 : 0,85 | E5 : 0,91 |
| D5 : 0,70 | E6 : 0,63 |
| D6 : 0,63 | E7 : 0,46 |
| D7 : 0,55 | E8 : 0,40 |
| D8 : 0,54 | E9 : 0,31 |
| D9 : 0,53 | E10: 0,28 |
| D10: 0,50 | |
| D11: 0,40 | |
| D12: 0,14 | |
| D13: 0,12 | |
| D14: $<$0,1 | |
| D15: $<$0,1 | |
| D16: $<$0,1 | |

## 5. Restriktionsverdauungen

Einzelverdauungen wurden in einem Gesamtvolumen von 50 bis 100 µl mit den von Herstellern empfohlenen Puffern durchgeführt. Um eine vollständige Verdauung der DNA zu gewährleisten, wurden die Proben über Nacht inkubiert.

Bei Doppel- und Mehrfachverdauungen wurden die entsprechenden Enzyme meist gleichzeitig zur DNA zugesetzt. Der Puffer bestand in diesen Fällen aus einer Lösung, die pro Liter 50 mMol Kochsalz, 5 mMol Tris-HCl (auf pH 7,5 eingestellt), 6 mMol Magnesiumchlorid, 6 mMol 2-Mercaptoethanol und 100 mg Rinderserumalbumin enthielt. Kontrollversuche zeigten, daß die Enzyme in diesem Puffer die gleichen Ergebnisse liefern wie in den von den Herstellern empfohlenen. Ausnahmen bildeten Enzyme, die einen überdurchschnittlich hohen Salzbedarf haben. In diesen Fällen wurde zunächst bei geringer Salzkonzentration mit dem einen Enzyme verdaut und erst nach Erhöhung der Salzkonzentration das zweite Enzym zugegeben.

## 6. Partielle Verdauungen mit Eco RI

2 µg Plasmid-DNA wurden mit 1 U des Restriktionsenzyms in einem Gesamtvolumen von 50 µl bei 37 °C inkubiert. Gleiche Ansätze wurden unterschiedlich lang inkubiert und die Reaktion zu den verschiedenen Zeiten durch zehnminütiges Erhitzen des Ansatzes auf 70 °C abgestoppt. Eine gelelektrophoretische Analyse des jeweiligen Bandenmusters zeigte, wie weit die Verdauung nach den definierten Zeiten fortgeschritten war.

## 7. Wachstumsbedingungen

Alle verwendteten Escherichia coli-Stämme wurden in L-Broth (10 g Bactro Trypton, 5 g Hefe-Extrakt und 5 g Kochsalz pro 1 Liter Wasser) gezüchtet. Die Methylomonas clara-Stämme wurden in einem der beiden folgenden Minimal-Medien gezüchtet :

M 36 :

1,5 % Methanol

0,1 % $H_3PO_4$

0,083 % $K_2SO_4$
0,018 % $Na_2SO_4 \times 10\, H_2O$
0,036 % $MgSO_4 \times 7\, H_2O$
0,004 % $CaCO_3$
0,015 % Citronensäure
0,005 % $(NH_4)_2Fe(SO_4)_2 \times 6\, H_2O$
80 µl/l Spurenelementelösung

V 135 L :
1,5 % Methanol
0,16 % $K_2SO_4$
0,06 % $MgSO_4 \times 7\, H_2O$
0,025 % $Na_2SO_4$
0,014 % $CaCO_3$
0,01 % $Fe_2(SO_4)_3$
0,2 % $H_3PO_4$
0,28 % $NH_3$ (25 %)
0,3 % $KNO_3$
0,3 % $NaHCO_3$
1 ml/l Spurenelementelösung

Spurenelmentelösung :
0,05 g/l $H_3BO_3$
0,01 g/l KJ
0,04 g/l $MnSO_4 \times 4\, H_2O$
0,04 g/l $ZnSO_4 \times 7\, H_2O$
0,02 g/l $(NH_4)_6Mo_7O_{24}$

### 8. Konstruktion der Hybridvektoren

Das Plasmid pBE 3 wurde mit dem Restriktionsenzym Eco RI partiell verdaut, so daß der Hauptteil des Plasmids nur einmal geschnitten wurde und in der linearisierten Form vorlag. pBR 322-DNA wurde mit Eco RI total verdaut und anschließend einer Behandlung mit alkalischer Phosphatase unterzogen. Die so vorbereiteten DNAs wurden zusammengegeben und mit T4-DNA-Ligase bei 14 °C über Nacht inkubiert. 50 µg dieser ligierten Mischung dienten sodann zur Transformation in den durch $CaCl_2$-Behandlung kompetent gemachten E. coli-Stamm HB 101.

Die Bakterien wurden auf L-Broth-Platten mit 20 µg/ml Tetracyclin ausgestrichen und über Nacht bebrütet. Resistente Kolonien wurden auf frische Platten überimpft und gut ausgewachsene Klone durch « single colony lysis » auf das Vorhandensein von Plasmid-DNA mit höherem Molekulargewicht als pBR 322 untersucht. Klone, die eine solche Plasmid-DNA enthielten, wurden schließlich in 100 ml L-Broth mit oder ohne Tetracyclin angezüchtet und nach Chloramphenicol-Stimulierung der Bakterien die Plasmid-DNA gewonnen.

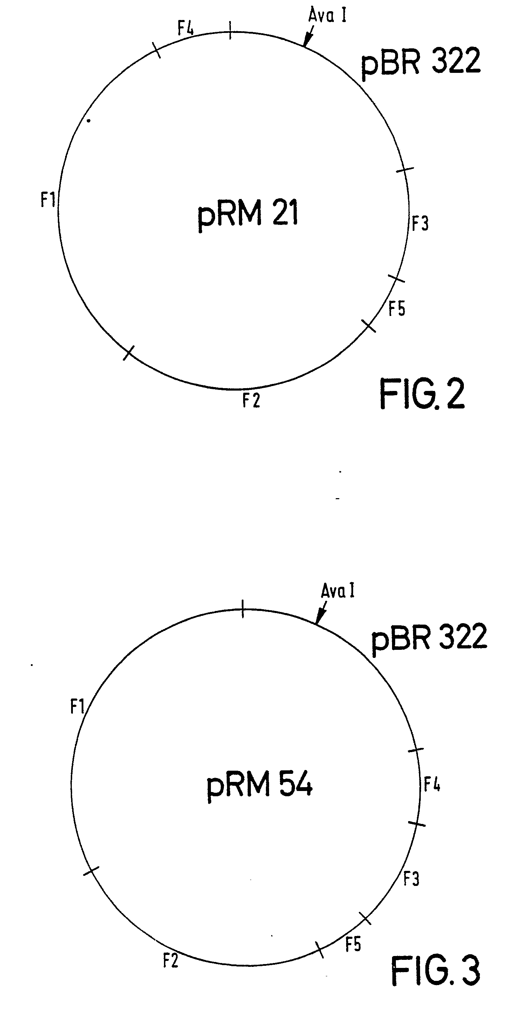

Restriktionsverdauungen mit den Enzymen Eco RI und AVA I zeigten, daß das in der Figur 2 gezeigte Hybridplasmid pRM 21 und das in der Figur 3 gezeigte Hybridplasmid pRM 54 erhalten worden waren.

Daneben können Hybridplasmide identifiziert werden, die Fraktionen von pBE 3 enthalten.

### 9. Ligase-Reaktion

Ligase-Reaktionen wurden in 50 µl Volumen bei einer Gesamt-DNA-Konzentration von 20 µg/ml ausgeführt. Der Puffer enthielt pro Liter 30 mMol Tris-HCl (auf pH 7,5 eingestellt), 4 mMol Magnesiumchlorid, 10 mMol Dithioerythrit und 0,2 mMol ATP. Zu diesen Ansätzen wurde 1 µl T4-Ligase entsprechend 400 U zugegeben (1 U entspricht hier der Menge Enzym, die nötig ist, um 50 % einer Hind III-verdauten lambda-DNA in 30 Minuten bei — 16 °C in einem Volumen von 20 µl zu ligieren. Die Konzentration der DNA in diesem Ansatz beträgt dabei etwa 330 µg/ml). Das dür ein bestimmtes Ligase-Experiment optimale Verhältnis der beiden DNAs zueinander wurde nach den Verfahren von Dugaiczyk et al., JMB 96 (1975) 171 erreichnet. Inkubiert wurde bei 14 °C für mindestens 16 Stunden.

### 10. Konjugation

Rezipient und Donor wurden über Nacht bis zu einer $OD_{600}$ von 1,0-1,2 (Rezipient) bzw. 1,4-1,6 (Donor) angezüchtet und gleiche Volumina von Rezipient und Donor so zusammen gegeben, daß der Mischung eine große Oberfläche zur Verfügung stand. Die Mischung wurde 2 Stunden lang ohne zu Schütteln bei 37 °C inkubiert und anschließend 200 µl davon auf Agarplatten ausgestrichen, deren Zusammensetzung eine Selektion gegen den Donor und für den mit einer neuen, durch das zu übertragende Plasmid vermittelten, Eigenschaft ausgestatteten Rezeptor erlaubte. Bei der Konjugation zwischen E. coli HB 101 und M. clara DSM 2397 waren dies Methanol-Minimalmedium-Platten mit Zusatz eines (durch die Art des zu übertragenden Plasmids bestimmten) Antibiotikums.

In anderen Experimenten wurden Rezipient und Donor wie oben beschrieben inkubiert und 200 µl dieser Mischung auf Methanol-Minimalmedium-Platten ohne Antibiotikum ausgestrichen. Diese Platten wurden über Nacht bei 37 °C bebrütet und der entstandene Bakterienrasen mit 2 ml Methanol-Minimalmedium abgeschwemmt. 200 µl dieser Suspension kamen dann wieder zum Ausstrich auf Methanol-Minimalmedium-Platten mit dem entsprechenden Antibiotikum. Klone zeigten sich nach 48-72 stündigem Bebrüten der Platten bei 37 °C.

11. Expression eukaryotischer DNA in M. clara

a) Das Plasmid pBE 3 wurde nach den beschriebenen Verfahren in Derivaten von pBR 322 kloniert bzw. umkloniert, die als c-DNA-Sequenz eines eukaryotischen Gens die des Affen-Insulins enthielten.

Affeninsulin-c-DNA ist in die Pst I-Schnittstelle von pBR 322 derart eingebaut worden, daß diese fremde Information unter der Kontrolle des β-Lactamase-Promotors in E. coli exprimiert wurde. Dabei entstand ein Fusionsprotein, das mit Antiinsulin-Antikörpern nachgewiesen werden konnte (Europäische Patentanmeldung 0 032 675).

Die Affeninsulin-c-DNA enthält zwei interne Pst I-Schnittstellen. Die kodierende Information kann daher durch Pst-I-Verdauung nicht in intakter Form aus dem Plasmid entfernt werden. Um eine Replikation dieses Plasmids in M. clara zu ermöglichen, wurde daher so vorgegangen, daß DNA-Sequenzen aus dem M. clara-Plasmid pBE 3 in dieses Insulininformation experimierende pBR 322-Derivat eingebaut wurde. Die experimentelle Durchführung erfolgte wie vorstehend beschrieben. Hierbei konnte eine Reihe von hybriden Plasmiden erhalten werden, die sich in ihrer Struktur nur durch die in den pBR-322-Anteil eingebaute Affeninsulin-c-DNA von den zuvor beschriebenen Hybridvektoren unterscheiden. Die Anordnung des Insulingens im pBR 322-Anteil dieser Vektoren bleibt dabei unverändert in Phase, so daß auch diese Klone Insulin-antigene Determinanten in E. coli exprimieren. Einer dieser Klone, der die gesamte pBE 3-Sequenz enthält, die Bezeichnung pInMc 68.

b) Der festgestellte Mobilisierbarkeitsdefekt von pBR 322 kann durch Plasmide wie Col K und Col V komplementiert werden (Young und Poulis, Gene 4 (1978) 175-179). Daher wurde in die oben beschriebenen, durch Calciumchlorid-Behandlung kompetent gemachten Donorstämme mit dem konjugativen Plasmid RP 4 und den Hybridvektoren der pRM-Reihe noch zusätzlich das Plasmid Col K durch Transformation eingeführt. Als Indikatorstamm zum Nachweis des Plasmids Col K wurde der Colicinempfindliches Stamm AB 1157 verwendet (Warren et al., MGG 170 (1979) 103-107). Um in Konjugationsexperimenten zwischen den E. coli-Donorstämmen HB 101 (RP 4, Col K, pRM 54) bzw. HB 101 (RP 4, Col K, pRM 21) und dem Rezipienten M. clara ATCC 31226 auf Klone zu selektionieren, die den Hybridvektor enthielten, wurde in Gegenwart hoher Dosen Tetracyclin (50 µg/ml) auf Methanol-Minimalmedium (M 36) selektioniert.

Eine Analyse zahlreicher M. clara-Klone ergab, daß in ca. 10 % der Fälle Klone erhalten worden waren, die nur den Hybridvektor enthielten. In einem Konjugationsexperiment zwischen dem E. coli-Donorstamm HB 101 (RP 4, Col K, InMc 68) und dem M. clara-Rezipienten ATCC 31226 konnten in gleicher Weise M. clara-Klone erhalten werden, die nur das Plasmid pInMc 68 enthielten.

c) Die erhaltenen M. clara-Klone mit dem Plasmid pInMc 68 wurden in der Folge gezüchtet und über einen Radioimmunassay bzw. einen Fettzellassay auf ihren Insulingehalt überprüft. Entspechend dem in der Europäischen Patentanmeldung 0 032 675 mit E. coli gemachten Beobachtungen konnten auch im Falle von M. clara ATCC 31226 Insulinwerte zwischen 1 und 5 IE pro Liter gemessen werden. Somit wird also die im pBR 322-Anteil des Hybridvektors pIncMc 68 enthaltene Insulininformation auch in M. clara korrekt und effizient exprimiert.

12. Der Stamm Merthylomonas clara ATCC 31226 wird in dem Minimal-Medium M 36 bis zu einer OD$_{600}$ von 0,9 bis 1,4 angezüchtet. 20 ml dieser Kulturen werden in 100 ml M 36-Medium eingebracht, das außerdem 4 % Glycin und 10 % Saccharose enthält. In diesem Medium werden die Bakterien 4 Stunden bei 37 °C und 100 bis 180 Upm geschüttelt. Unter dem Phasenkontrastmikroskop sind dann keine länglichen, beweglichen Bakterien mehr zu erkennen.

Die gebildeten Sphäroplasten werden bei 4 °C 10 Minuten bei 2 600 g abzentrifugiert und in 1 ml M 36-Medium resuspendiert, das zusätzlich 10 % Saccharose enthält.

Die genannten Medien können anstelle der 10 % Saccharose auch 1 Mol pro Liter Sorbit oder 15 % Polyethylenglykol vom mittleren Molgewicht 6 000 enthalten.

Die Abtrennung der Sphäroplasten kann auch durch 10-minütiges Zentrifugieren bei 4 °C und 3 600 g erfolgen.

13. 0,5 ml der nach Beispiel 12 erhaltenen Suspension werden mit 0,1 ml einer Mischung versetzt, die zu gleichen Teilen aus Resuspensionsmedium und TE-Puffer besteht, in dem das Plasmid pRM 21 enthalten ist. Zu dieser Mischung gibt man 1,5 ml 4 %ige Polyethylenglykol-6 000-Lösung und durchmischt vorsichtig. Diese Mischung läßt man 2 Minuten bei Raumtemperatur stehen, gibt 5 ml Resuspensionsmedium zu und zentrifugiert bei 3 600 g.

Der Niederschlag wird in 1 ml Resuspensionsmedium aufgenommen und auf Agarplatten ausgestrichen die in ihrer Zusammensetzung dem Resuspensionsmedium entsprechen und zusätzlich 1,5 % Bakto-Agar und 50 µg/ml Ampicillin enthalten. Die Platten werden bie 37 °C 3 Tage bebrütet und die Kolonien in M 36-Medium, das 50 µg/ml Ampicillin enthält, gezüchtet. Aus den so erhaltenen Bakterien konnte das zur Transformation eingesetzte Plamid pRM 21 isoliert werden.

Anstelle von Ampicillin kann bei diesem Verfahren auch jeweils Tetracyclin in einer Konzentration von 10 μl/ml eingesetzt werden.

Das gleiche Ergebnis erhält man auch, wenn man anstelle des genannten Plasmids das Plasmid pRM 54 einsetzt.

14. Arbeitet man nach Beispiel 13, setzt jedoch als Plasmid pINMc 68 ein, und selektioniert mit 10 μg/ml Tetracyclin, so erhält man Methylomonas clara-Klone mit diesem Plasmid, die Insulin produzieren.

**Patentansprüche** (für die Vertragsstaaten : BE, CH, DE, FR, GB, IT, LI, LU, NL, SE)

1. Hybridplasmide mit einem einem obligat methylotrophen Bakterium eigenen Plasmid-Replicon.

2. Hybridplasmid nach Anspruch 1, das in einem Bakterium der Gattung Methylomonas, vorzugsweise der Art Methylomonas clara, insbesondere von Stamm ATCC 31 226 repliziert wird.

3. Hybridplasmid nach Anspruch 1 oder 2 mit integrierter eukaryotischer DNA, insbesondere für die Expression von Insulin.

4. Hybridplasmid nach Anspruch 1 oder 2 mit integrierter prokaryotischer DNA.

5. Verfahren zur Herstellung von obligat methylotrophen Bakterien, die enthaltene Fremd-DNA exprimieren, dadurch gekennzeichnet, daß man

a) aus einem obligat methylotrophen Bakterium ein Plasmid isoliert,

b) hieraus und aus einem Plasmid mit Selektionsmarkern ein Hybridplasmid mit einem dem obligat methylotrophen Bakterium eigenen Replicon herstellt,

c) dieses Hybridplasmid durch Transformation in einen Wirtsorganismus einbringt und dort amplifiziert,

d) nach Selektion die Klone mit einem geeigneten konjugativen Plasmid behandelt und den Mobilisierbarkeitsdefekt behebt,

e) die so erhaltenen Klone mit vorzugsweise plasmidfreien obligat methylotrophen Bakterien als Rezipient konjugiert und

f) die gewünschten Klone selektioniert.

6. Verfahren nach Anspruch 5, dadurch gekennzeichnet, daß das als Rezipient dienende obligat methylotrophe Bakterium eines der Gattung Methylomonas, vorzugsweise von der Art Methylomonas clara, insbesondere vom Stamm ATCC 31 226 ist.

7. Verfahren nach Anspruch 5 oder 6, dadurch gekennzeichnet, daß das Bakterium, aus dem das Plasmid isoliert wird, vom Stamm Methylomonas clara DSM 2 397 ist.

8. Verfahren nach einem oder mehreren der Ansprüche 5 bis 7, dadurch gekennzeichnet, daß das Plasmid mit Selektionsmarkern pBR 322 ist und gegebenenfalls die genetische Information für die Expression von Insulin enthält.

9. Verfahren nach einem oder mehreren der Ansprüche 5 bis 8, dadurch gekennzeichnet, daß der Wirtsorganismus Escherichia coli ist, das konjugative Plasmid RP 4 ist und der Mobilisierbarkeitsdefekt durch Einführung des Plasmids Col K oder Col V behoben wird.

10. Verfahren nach einem oder mehreren der Ansprüche 5 bis 8, dadurch gekennzeichnet, daß man anstelle der Verfahrensschritte d) und e) das als Rezipient dienende obligat methylotrophe Bakterium in die Sphäroplasten überführt und das Hybridplasmid durch Transformation einbringt.

11. Vefahren zur Herstellung der in Anspruch 10 genannten Sphäroplasten aus obligat methylotrophen Bakterien, dadurch gekennzeichnet, daß man die Bakterien in einem glycinreichen Medium, das einen osmotischen Stabilisator enthält, züchtet, wobei das Medium vorzugsweise schwach hypotonisch ist und 2 bis 4 Gew.-% Glycin enthält.

12. Plasmide aus Methylomonas clara DSM 2 397.

13. Wirtsorganismus, enthaltend ein Plasmid nach einem oder mehreren der Ansprüche 1 bis 5, sowie gegebenenfalls ein konjugatives Plasmid und gegebenenfalls ein den Mobilisierbarkeitsdefekt behebendes Plasmid.

14. Organismus nach Anspruch 13, der als konjugatives Plasmid RP 4 sowie gegebenenfalls das Plasmid Col K oder Col V enthält.

15. Sphäroplasten aus obligat methylotrophen Bakterien, insbesondere der Gattung Methylomonas, vor allem der Art Methylomonas clara, speziell vom Stamm Methylomonas clara ATCC 31 226, erhältlich nach dem Verfahren gemäß Anspruch 11.

**Patentansprüche** (für den Vertragsstaaten AT)

1. Verfahren zur Herstellung von obligat methylotrophen Bakterien, die enthaltene Fremd-DNA exprimieren, dadurch gekennzeichnet, daß man

a) aus einem obligat methylotrophen Bakterium ein Plasmid isoliert,

b) hieraus und aus einem Plasmid mit Selektionsmarkern ein Hybridplasmid mit einem dem obligat methylotrophen Bakterium eigenen Replicon herstellt,

c) dieses Hybridplasmid durch Transformation in einen Wirtsorganismus einbringt und dort amplifiziert,

d) nach Selektion die Klone mit einem geeigneten konjugativen Plasmid behandelt und den Mobilisierbarkeitsdefekt behebt,

e) die so erhaltenen Klone mit vorzugsweise plasmid-freien obligat methylotrophen Bakterien als Rezipient konjugiert und

f) die gewünschten Klone selektioniert.

2. Verfahren zur Herstellung des in Anspruch 1 genannten Hybridplasmids, dadurch gekennzeichnet, daß man aus einem obligat methylotrophen Bakterium ein Plasmid isoliert und hieraus und aus einem Plasmid mit Selektionsmarkern ein Hybridplasmid mit einem dem obligat methylotrophen Bakterium eigenen Replicon herstellt.

3. Verfahren nach Anspruch 1 oder 2, dadurch gekennzeichnet, daß das als Rezipient dienende obligat methylotrophe Bakterium eines der Gattung Methylomonas, vorzugsweise der Art Methylomonas clara, insbesondere vom Stamm ATCC 31 226 ist.

4. Verfahren nach einem oder mehreren der Ansprüche 1 bis 3, dadurch gekennzeichnet, daß das Bakterium, aus dem das Plasmid isoliert wird, vom Stamm Methylomonas clara DSM 2 397 ist.

5. Verfahren nach einem oder mehreren der Ansprüche 1, 3 oder 4, dadurch gekennzeichnet, daß das Plasmid mit Selektionsmarkern pBR 322 ist und gegebenenfalls die genetische Information für die Expression von Insulin enthält.

6. Verfahren nach einem oder mehreren der Ansprüche 1 oder 3 bis 5, dadurch gekennzeichnet, daß der Wirtsorganismus Escherichia coli ist, das konjugative Plasmid RP 4 ist und der Mobilisierbarkeitsdefekt durch Einführung des Plasmids Col K oder Col V behoben wird.

7. Verfahren nach einem oder mehreren der Ansprüche 1 oder 3 bis 6, dadurch gekennzeichnet, daß man anstelle der Verfahrensschritte d) und e) das als Rezipient dienende obligat methylotrophe Bakterium in die Sphäroplasten überführt und das Hybridplasmid durch Transformation einbringt.

8. Verfahren zur Herstellung der in Anspruch 7 genannten Sphäroplasten aus obligat methylotrophen Bakterien, dadurch gekennzeichnet, daß man die Bakterien in einem glycinreichen Medium, das einen osmotischen Stabilisator enthält, züchtet, wobei das Medium vorzugsweise schwach hypotonisch ist und 2 bis 4 Gew.-% Glycin enthält.

## Claims (for the Contracting States : BE, CH, DE, FR, GB, IT, LI, LU, NL, SE)

1. A hybrid plasmid with a plasmid replicon inherent to an obligate methylotrophic bacterium.

2. A hybrid plasmid as claimed in claim 1, which is replicated in a bacterium of the genus Methylomonas, preferably of the species Methylomonas clara, in particular of the strain ATCC 31 226.

3. A hybrid plasmid as claimed in claim 1 or 2, having integrated eukaryotic DNA, in particular for the expression of insulin.

4. A hybrid plasmid as claimed in claim 1 or 2 having integrated prokaryotic DNA.

5. A process for the preparation of obligate methylotrophic bacteria, which express contained foreign DNA, which comprises

a) isolating a plasmid from an obligate methylotrophic bacterium,

b) preparing from this and from a plasmid with selection markers a hybrid plasmid with a replicon inherent to the obligate methylotrophic bacterium,

c) introducing this hybrid plasmid by transformation into a host organism and amplifying it there,

d) after selection, treating the clones with a suitable conjugative plasmid and abolishing the mobilizability defect,

e) conjugating the clones thus obtained with, preferably plasmid-free, obligate methylotrophic bacteria as recipient and

f) selecting the desired clones.

6. The process as claimed in claim 5, wherein the obligate methylotrophic bacterium serving as recipient is of the genus Methylomonas, preferably of the species Methylomonas clara, in particular of the strain ATCC 31 226.

7. The process as claimed in claim 5 or 6, wherein the bacterium from which the plasmid is isolated is of the strain Methylomonas clara DSM 2 397.

8. The process as claimed in one or more of claims 5 to 7, wherein the plasmid having selection markers is pBR 322 and, where appropriate, contains the genetic information for the expression of insulin.

9. The process as claimed in one or more of claims 5 to 8, wherein the host organism is Escherichia coli, the conjugative plasmid is RP 4, and the mobilizability defect is abolished by introduction of the plasmid Col K or Col V.

10. The process as claimed in one or more of claims 5 to 8, wherein, in place of the process steps d) and e), the obligate methylotrophic bacterium serving as recipient is converted into spheroplasts, and the hybrid plasmid is inserted by transformation.

11. A process for the preparation of the spheroplasts of obligate methylotrophic bacteria mentioned in claim 10, which comprises culturing the bacteria in a glycinerich medium containing an osmotic stabilizer, the medium preferably being slightly hypotonic and containing 2 to 4 % by weight of glycine.

12. Plasmids from Methylomonas clara DSM 2 397.

13. A host organism containing a plasmid as claimed in one or more of claims 1 to 5 and, where appropriate, a conjugative plasmid and, where appropriate, a plasmid abolishing the mobilizability defect.

14. An organism as claimed in claim 13, which contains RP 4 as the conjugative plasmid and, where appropriate, the plasmid Col K or Col V.

15. Spheroplasts of obligate methylotrophic bacteria, in particular of the genus Methylomonas, especially of the species Methylomonas clara, specifically of the strain Methylomonas clara ATCC 31 226, obtainable by the process as claimed in claim 11.

**Claims** (for the Contracting State AT)

1. A process for the preparation of obligate methylotrophic bacteria, which express contained foreign DNA, which comprises

a) isolating a plasmid from an obligate methylotrophic bacterium,

b) preparing from this and from a plasmid with selection markers a hybrid plasmid with a replicon inherent to the obligate methylotrophic bacterium,

c) introducing this hybrid plasmid by transformation into a host organism and amplifying it there,

d) after selection, treating the clones with a suitable conjugative plasmid and abolishing the mobilizability defect,

e) conjugating the clones thus obtained with, preferably plasmid-free, obligate methylotrophic bacteria as recipient and

f) selecting the desired clones.

2. A process for the preparation of the hybrid plasmid mentioned in claim 1, which comprises isolating a plasmid from an obligate methylotrophic bacterium, and preparing from this and from a plasmid with selection markers a hybrid plasmid with a replicon inherent to the obligate methylotrophic bacterium.

3. The process as claimed in claim 1 or 2, wherein the obligate methylotrophic bacterium serving as recipient is of the genus Methylomonas, preferably of the species Methylomonas clara, in particular of the strain ATCC 31 226.

4. The process as claimed in one or more of claims 1 to 3, wherein the bacterium from which the plasmid is isolated is of the strain Methylomonas clara DSM 2 397.

5. The process as claimed in one or more of claims 1, 3 or 4, wherein the plasmid having selection markers is pBR 322 and, where appropriate, contains the genetic information for the expression of insulin.

6. The process as claimed in one or more of claims 1 or 3 to 5, wherein the host organism is Escherichia coli, the conjugative plasmid is RP 4, and the mobilizability defect is abolished by introduction of the plasmid Col K or Col V.

7. The process as claimed in one or more of claims 1 or 3 to 6, wherein, in place of the process steps d) and e), the obligate methylotrophic bacterium serving as recipient is converted into spheroplasts, and the hybrid plasmid is inserted by transformation.

8. A process for the preparation of the spheroplasts of obligate methylotrophic bacteria mentioned in claim 7, which comprises culturing the bacteria in a glycine-rich medium containing an osmotic stabilizer, the medium preferably being slightly hypotonic and containing 2 to 4 % by weight of glycine.

**Revendications** (pour les Etats contractants : BE, CH, DE, FR, GB, IT, LI, LU, NL, SE)

1. Plasmides hybrides comportant un réplicon plasmidique propre à une bactérie méthylotrophe stricte.

2. Plasmide hybride selon la revendication 1, qui est répliqué dans une bactérie appartenant au genre Methylomonas, de préférence à l'espèce Methylomonas clara, en particulier à la souche ATCC 31 226.

3. Plasmide hybride selon la revendication 1 ou 2, comportant de l'ADN intégré d'eucaryote, en particulier pour l'expression de l'insuline.

4. Plasmide hybride selon la revendication 1 ou 2, comportant de l'ADN intégré de procaryote.

5. Procédé pour l'obtention de bactéries méthylotrophes strictes qui expriment de l'ADN étranger contenu, caractérisé en ce que

a) on isole un plasmide provenant d'une bactérie méthylotrophe stricte,

b) à partir de celui-ci et à partir d'un plasmide comportant des marqueurs sélectifs, on construit un plasmide hybride comportant un réplicon propre à la bactérie méthylotrophe stricte,

c) ce plasmide hybride est introduit par transformation dans un organisme hôte et s'y amplifie,

d) après sélection, on traite les clones avec un plasmide conjugant approprié et on élimine la déficience d'aptitude à la mobilisation du plasmide hybride,

e) on fait conjuguer les clones ainsi obtenus avec des bactéries méthylotrophes strictes, de préférence exemptes de plasmides, en tant que récepteurs, et

f) on sélectionne les clones recherchés.

6. Procédé selon la revendication 5, caractérisé en ce que la bactérie méthylotrophe stricte utilisée en tant que récepteur est une bactérie appartenant au genre Methylomonas, de préférence à l'espèce Methylomonas clara, en particulier à la souche ATCC 31 226.

7. Procédé selon la revendication 5 ou 6, caractérisé en ce que la bactérie à pratir de laquelle est isolé le plasmide appartient à la souche Methylomonas clara DSM 2 397.

8. Procédé selon une ou plusieurs des revendications 5 à 8 caractérisé en ce que le plasmide comportant des marqueurs sélectifs est pBR 322 et éventuellement contient l'information génétique pour l'expression de l'insuline.

9. Procédé selon une ou plusieurs des revendications 5 à 8, caractérisé en ce que l'organisme hôte est Escherichia coli, le plasmide conjugant est RP 4 et la déficience d'aptitude à la mobilisation est éliminée par introduction du plasmide Col K ou Col V.

10. Procédé selon une ou plusieurs des revendications 5 ou 8, caractérisé en ce que, au lieu des étapes d) et e) du procédé, on transforme en les sphéroplastes la bactérie méthylotrophe stricte utilisée en tant que récepteur, et on introduit par transformation le plasmide hybride.

11. Procédé pour la préparation des sphéroplastes cités dans la revendication 10, à partir de bactéries méthylotrophes strictes, caractérisé en ce que l'on cultive les bactéries dans un milieu riche en glycine, qui contient un stabilisant osmotique, le milieu de préférence étant légèrement hypotonique et contenant de 2 à 4 % en poids de glycine.

12. Plasmides obtenus à partir de Methylomonas clara DSM 2 397.

13. Organismes hôtes contenant un plasmide selon une ou plusieurs des revendications 1 à 5, ainsi qu'éventuellement un plasmide conjugant et éventuellement un plasmide éliminant la déficience d'aptitude à la mobilisation.

14. Organisme selon la revendication 13, qui contient RP 4 en tant que plasmide conjugant, ainsi qu'éventuellement le plasmide Col K ou Col V.

15. Sphéroplastes provenant de bactéries méthylotrophes strictes, appartenant notamment au genre Methylomonas, en particulier à l'espèce Methylomonas clara, de préférence à la souche Methylomonas clara ATCC 31 226, pouvant être obtenus conformément au procédé selon la revendication 11.

**Revendications** (pour l'Etat contractant AT)

1. Procédé pour l'obtention de bactéries méthylotrophes strictes qui expriment de l'ADN étranger contenu, caractérisé en ce que

a) on isole un plasmide provenant d'une bactérie méthylotrophe stricte,

b) à partir de celui-ci et à partir d'un plasmide comportant des marqueurs sélectifs, on construit un plasmide hybride comportant un réplicon propre à la bactérie méthylotrophe stricte,

c) ce plasmide hybride est introduit par transformation dans un organisme hôte et s'y amplifie,

d) après sélection, on traite les clones avec un plasmide conjugant approprié et on élimine la déficience d'aptitude à la mobilisation du plasmide hybride,

e) on fait conjuguer les clones ainsi obtenus avec des bactéries méthylotrophes strictes, de préférence exemptes de plasmides, en tant que récepteurs, et

f) on sélectionne les clones recherchés.

2. Procédé pour la construction du plasmide hybride cité dans la revendication 1, caractérisé en ce que l'on isole un plasmide à partir d'une bactérie méthylotrophe stricte, et, à partir de celui-ci et d'un plasmide comportant des marqueurs sélectifs, on construit un plasmide hybride comportant un réplicon propre à la bactérie méthylotrophe stricte.

3. Procédé selon la revendication 1 ou 2, caractérisé en ce que la bactérie méthylotrophe stricte utilisée en tant que récepteur est une bactérie appartenant au genre Methylomonas, de préférence à l'espèce Methylomonas clara, en particulier à la souche ATCC 31 226.

4. Procédé selon une ou plusieurs des revendications 1 à 3, caractérisé en ce que la bactérie à partir de laquelle est isolé le plasmide appartient à la souche Methylomonas clara DSM 2 397.

5. Procédé selon une ou plusieurs des revendications 1, 3 ou 4, caractérisé en ce que le plasmide comportant des marqueurs sélectifs est pBR 322 et éventuellement contient l'information génétique pour l'expression de l'insuline.

6. Procédé selon une ou plusieurs des revendications 1 ou 3 à 5, caractérisé en ce que l'organisme hôte est Escherichia coli, le plasmide conjugant est RP 4 et la déficience d'aptitude à la mobilisation est éliminée par introduction du plasmide Col K ou Col V.

7. Procédé selon une ou plusieurs des revendication 1 ou 3 à 6, caractérisé en ce que, au lieu des étapes d) et e) du procédé, on transforme en les sphéroplastes la bactérie méthylotrophe stricte utilisée en tant que récepteur, et on introduit par transformation le plasmide hybride.

8. Procédé pour la préparation des sphéroplastes cités dans la revendication 7, à partir de bactéries méthylotrophes strictes, caractérisé en ce que l'on cultive les bactéries dans un milieu riche en glycine, qui contient un stabilisant osmotique, le milieu de préférence étant légèrement hypotonique et contenant de 2 à 4 % en poids de glycine.

# FIG.1

pBR 322

pRM 21

F4

Ava I

F1

F3

F5

F2

FIG.2

pBR 322

pRM 54

Ava I

F1

F4

F3

F5

F2

FIG.3